# EUROPÄISCHE PATENTSCHRIFT

(11) **EP 1 408 914 B1**
(45) Veröffentlichungstag und Bekanntmachung des Hinweises auf die Patenterteilung: **12.09.2012**
(21) Anmeldenummer: 02764722.1
(22) Anmeldetag: 18.07.2002
(51) Int. Cl.: A61Q 1/02, A61Q 1/04, A61Q 1/08, A61Q 1/10, A61K 8/31, A61K 8/81

(54) **WASSER-UND ABRIEBBESTÄNDIGES KOSMETIKUM FÜR HAARE UND DEKORATIVE KOSMETIK**
WATER-RESISTANT AND ABRASION-RESISTANT HAIR COSMETIC AND DECORATIVE COSMETIC
PRODUIT COSMETIQUE CAPILLAIRE RESISTANT A L'EAU ET A L'ABRASION ET AYANT UN EFFET DECORATIF

(30) Priorität: 24.07.2001 DE 10136883
(43) Veröffentlichungstag der Anmeldung: 21.04.2004
(73) Patentinhaber: Coty B.V., 2031 CC Haarlem (NL)
(72) Erfinder: LOGINOVA, Yelena, Bronx, NY 10467 (US); CERNASOV, Domnica, Ringwood, NJ 07456 (US); MACCHIO, Ralph, Sparta, NJ 07971 (US)
(74) Vertreter: Ziebig, Marlene
(86) Internationale Anmeldenummer: PCT/EP2002/008006
(87) Internationale Veröffentlichungsnummer: WO 2003/009821

(56) Entgegenhaltungen:
- WO-A-00/56280
- WO-A-98/18431
- GB-A- 1 110 240
- US-A- 4 423 031

## Beschreibung

Die Erfindung betrifft ein kosmetisches Produkt für Haare und für die dekorative Kosmetik gemäß Anspruch 1, das besonders wasser- und abriebbeständig ist.

Aus der US-A-5925337 ist eine wasserfeste Mascara-Zusammensetzung bekannt, die 2-40 Gew-% eines Wachses, 5-15 Gew-% eines Verdickungsmittels, 35-50 Gew-% eines flüchtigen organischen Lösungsmittels und 1-35 Gew-% eines wasserlöslichen filmbildenden Mittels enthält, wobei letzteres z.B. auch ein Acrylatpolymer sein kann. Die Zusammensetzung enthält keinen Emulgator.

Der Erfindung liegt die Aufgabe zugrunde, ein neues kosmetisches Produkt für die dekorative Kosmetik und für die Haare bereitzustellen, das auf Grund seiner besonderen Struktur geeignet ist, ein weiches und elastisches Produkt mit verbesserten wasserfesten und abriebfesten Eigenschaften zu bilden, insgesamt mit einem verbesserten Nutzungskomfort.

Erfindungsgemäß ist das kosmetische Produkt für die Haare und die dekorative Kosmetik dadurch gekennzeichnet, daß es enthält - 1-45 Gew-% eines stabilen Gemisches aus einem mit Wasser emulgierbaren, filmbildenden Mittels auf Basis eines Acrylatcopolymeren oder von Acryl- oder Methacrylsäure oder eines Esters davon, eines mit Wasser nicht mischbaren aliphatischen Kohlenwasserstoff-Lösungsmittels, eines nicht-ionischen Emulgators und Wasser;
- 1-45 Gew-% eines kosmetischen Wachses;
- 0,1-10 Gew-% eines in einem Lösungsmittel dispergierbaren rheologischen Modifiziermittels;
- 0,1-30 Gew-% einer Pigmentphase, umfassend mineralische Pigmente, organische Pigmente, Perlglanzpigmente, oberflächenbehandelte Pigmente und Gemische davon;
- 51-90 Gew-% eines flüchtigen organischen Lösungsmittels;
- 1-15 Gew-% eines öllöslichen polymeren Filmbildners;
- 0,01-30 Gew-% eines Siliconharzes;
- 0,01-20 Gew-% eines Füllstoffes;
wobei alle Angaben auf das Gesamtgewicht des Produktes bezogen sind.

Das stabile Gemisch mit dem Acrylatcopolymeren, dem Kohlenwasserstoff-Lösungsmittel, dem Emulgator und Wasser besteht aus 0,01-30 Gew-% eines mit Wasser emulgierbaren, filmbildenden Mittels auf Basis eines Acrylatcopolymeren oder Acrylatderivates; 0,01-35 Gew-% eines aliphatischen Kohlenwasserstoff-Lösungsmittels oder eines flüchtigen Siliconderivates, wobei beide mit Wasser nicht mischbar sind und mit auf Wasser basierenden Ingredienzen und/oder auf organischen Lösungsmitteln basierenden Ingredienzen in Gegenwart eines Emulgators emulgierbar sind; 0,01 - 2 Gew-% eines nicht-ionischen Emulgators; und 0,1 - 30 Gew-% Wasser ; und wobei diese Zusammensetzung die Struktur eines Gels hat und wobei die Prozentangaben auf das Gesamtgewicht des Produktes bezogen sind.

Als Emulgator können vorteilhaft alkoxylierte Alkohole, ethoxylierte Alkohole, Polyglycerinester und Gemische davon verwendet werden. Dabei handelt es sich im nicht-ionische Emulgatoren, die als hydrophile Gruppe z.B. eine Polyolgruppe, eine Polyalkylglycolethergruppe oder eine Kombination davon enthält, wie z.B. Anlagerungsprodukte von 2-30 Mol Ethylenoxid an lineare Fettalkohole mit 8-22 Kohlenstoffatomen und an Fettsäuren mit 12-22 Kohlenstoffatomen. Weiterhin gehören dazu Polyglycerinester wie z.B. Polyglycerinricinoleat, oder Polyglycerinpoly-12-hydroxystearat sowie Gemische dieser verschiedene Substanzklassen.

Ein bevorzugter Emulgator ist beispielsweise Laureth-20, Laureth-23, Oleth-20, Steareth-20, Steareth-50, Ceteareth-20, Ceteareth-30 oder Ceteareth-50.

Als Acrylatcopolymer kann z.B. ein Ethylacrylat/Methylmethacrylat-Copolymeres eingesetzt werden oder ein Polymeres, hergestellt aus n-Butylacrylat, tert-Butylacrylat und Ethyl - oder Butylmethacrylat. Ein Handelsprodukt ist beispielsweise Daitosol 5000AD.

Besonders bevorzugt wird, wenn das filmbildende Mittel ein Ethylacrylat/Methylmethacrylat-Copolymeres mit einem Verhältnis vom Ethylacrylat-Einheiten zu Methylmethacrylat-Einheiten in dem Polymeren im Bereich von 7,5-8,5 : 1,8-2,3 ist.

Acrylsäure, Methacrylsäure oder deren einfache Ester können ebenfalls eingesetzt werden (hier auch als Acrylderivate bezeichnet).

Ein bevorzugter Bereich für das Acrylatcopolymere oder Acrylatderivat liegt zwischen 0,5 und 46 Gew-%, insbesondere 0,25-23 Gew-%, wobei alle Angaben auf das Gesamtgewicht des kosmetischen Produktes bezogen sind. Der Bereich für das Molekulargewicht der Acrylestermonomeren liegt bei 86 bis 128 und für die Methacrylate bei 100,11 bis 142,19.

In dem stabilen Gemisch mit Gelstruktur werden als aliphatisches Kohlenwasserstoff-Lösungsmittel, das mit Wasser nicht mischbar ist, vorzugsweise Isoparaffine (z.B. Isododecan, das besonders bevorzugt ist), Pentan, Hexan, Decan usw. oder spezielle Erdöldestillate verwendet.

Ein bevorzugter Bereich für das aliphatische Kohlenwasserstoff-Lösungsmittel oder das flüchtige Siliconderivat in dem stabilen Gemisch mit Gelstruktur liegt zwischen 55 und 90 Gew-%, bezogen auf das Gesamtgewicht des kosmetischen Produktes.

Als flüchtiges Siliconderivat kann vorteilhaft Cyclopentasiloxan oder Trimethylsiloxysilicat oder ein Gemisch davon eingesetzt werden. Dimethicone und Trimethylsiloxysilicat sind ebenfalls bevorzugte Siliconderivate.

Ein bevorzugtes Siliconderivat ist z.B. Decamethylcyclopentasiloxysiloxan (Silicone SS4230).

Der bevorzugte Anteil von Wasser liegt im Bereich von 1,0 bis 40 Gew-%, insbesondere 1,0 bis 20 Gew-%, bezogen auf das Gesamtgewicht des kosmetischen Produktes.

Als Wachse können eingesetzt werden natürliche pflanzliche Wachse, tierische Wachse, natürliche und synthetische Mineralwachse und synthetische Wachse. Dazu gehören beispielsweise Carnaubawachs, Candellilawachs, Bienenwachs, Wollwachs, Hartparaffin, Ceresin, Ozokerit, Mikrowachse, Silicone, Polyethylenglycol- oder -glycolesterwachse.

Pigmente, Pigmentgemische oder Pulver mit pigmentartiger Wirkung, worunter auch solche mit Perlglanz-Effekt zu verstehen sind, können zum Beispiel umfassen Eisenoxide, natürliche Aluminiumsilicate wie Ocker, Titan(di)oxid, Glimmer, Kaolin, manganhaltige Tone wie Umbra und roter Bolus, Calciumcarbonat, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, verkapselte und unverkapselte Getreidestärken sowie Glimmer-Titanoxid-organischer Farbstoff und andere oberflächenbehandelte Partikel.

Ein in einem Lösungsmittel dispergierbares rheologisches Modifikationsmittell, das auch als Stabilisator bezeichnet werden kann, ist beispielsweise ein organisch modifizierter Ton wie Disteardimonium Hectorite oder Steralkonium Hectorite.

Als flüchtiges organisches Lösungsmittel kann ein Kohlenwasserstoff-Lösungsmittel eingesetzt werde, wie es bereits oben beschrieben wurde, oder ein flüchtiges Siliconderivat.

Ein löslicher polymerer Filmbildner wird z.B. ausgewählt unter Copolymeren von Vinylpyrrolidon und langkettigen α-Olefinen, wie PVP/Eicosene Copolymer.

Füllstoffe sind beispielsweise Talkum, Stärke (behandelt oder unbehandelt), Kaolin und Polyamid; auch bestimmte Hilfsstoffe können dazugehören, wie Wasser, Konservierungsmittel, Vitamine, Farbstoffe, Radikalfänger, Antioxidationsmittel, Duftstoffe, Erweichungsmittel, Sequestriermittel, Bindemittel, pH-Regulatoren, Aminosäuren, Proteine, polare und unpolare öle oder auch anorganische und organische Lichtschutzmittel.

Das stabile Gemisch aus dem Acrylatcopolymeren, dem Kohlenwasserstoff-Lösungsmittel, dem Emulgator und Wasser ist ein Gel, das sich besonders dadurch auszeichnet, daß eine in der kosmetischen Formulierung stabile Kombination von Lösungsmittel und Filmbildner erhalten wird. Eine solche stabile Kombination mit Gelcharakter war für den Fachmann überraschend, und sie gestattet die Herstellung von kosmetischen Produkten mit besonders hoher Elastizität und Weichheit, wie sie die erfindungsgemäßen Produkte darstellen. So läßt sich beispielsweise die Elastizität auf das 4-5-fache steigern gegenüber der Elastizität des eingesetzten Filmbildners, z.B. bei einem Ethylacrylat/Methylmethacrylat-Copolymer.

Die Elastizität wurde ermittelt durch die Teststreifen-Methode, die folgendermaßen durchgeführt wird:
Es wird die Länge eines aus dem Polymeren hergestellten elastischen Filmes gemessen. Auf eine nichtklebrigen Oberfläche werden separat zwei Filme A und B von etwa 150 µm (6,0 mil) Dicke mittels eines Bird-Applikators gegossen. Film A besteht aus einem wäßrigen Acrylatcopolymeren (Acrylates Copolymer : Wasser etwa 50:50, Emulgator), Film B besteht aus 50 % Bestandteile A und 50 % Isododecane. Nach 24 Stunden bei 25 °C werden Probestreifen von etwa 50x25 mm aus beiden Filmen herausgeschnitten. Ein Ende des Films wird an einem Lineal eingespannt. Die Proben werden in Richtung auf das Linealende bis zum Abriß gezogen. Das Ergebnis für Film A betrug 101 mm, für Film B 406 mm.

Auch die Weichheit der Zusammensetzung nach dem Auftragen auf die Haut oder auf einen ebene Fläche zeigt sich deutlich erhöht gegenüber dem ursprünglichen Filmbildner. Dadurch verbessert sich das Hautgefühl (feeling), das eine wesentliche Rolle spielt bei der Produktwahl durch den Anwender.

Die Herstellung des stabilen Gels mit Acrylatcopolymeren, Kohlenwasserstoff-Lösungsmittel, Emulgator und Wasser erfolgt in der Weise, daß
ein Gemisch eines filmbildenden Mittels auf Basis eines Acrylatcopolymeren oder Acrylatderivates, Wasser und eines nicht-ionischen Emulgators auf eine Temperatur im Bereich von 45 bis 50 °C erwärmt wird;
die erhaltene Emulsion bei dieser Temperatur mit einem flüchtigen aliphatischen Kohlenwasserstoff-Lösungsmittels oder einem flüchtigen Siliconderivat vermischt wird, wobei das Lösungsmittel oder das Siliconderivat mit Wasser nicht mischbar sind, jedoch mit auf Wasser basierenden Ingredienzen und/- oder mit auf organischen Lösungsmitteln basierenden Ingredienzen in Gegenwart eines Emulgators emulgierbar sind, und wobei nach Homogenisierung bei 1500-3000 U/min für 15 bis 60 Minuten ein zweiphasiges flüssiges System erhalten wird bei vollständiger Verteilung der mit Wasser nicht mischbaren organischen Phase als Mikrotröpfchen in der wäßrigen Phase; und
das Gemisch auf 25-30 °C abgekühlt wird unter Rühren mit einer Geschwindigkeit von 300 bis 600 U/min.

Anschließend erfolgt die Verarbeitung mit den anderen kosmetischen Hilfs- und/oder Wirkstoffen in üblicher Weise bei Temperaturen von etwa 35 bis 40 °C.

Das Verfahren zeichnet sich durch eine genau vorgegebene Abfolge von Schritten sowie ein unübliches Temperaturregime aus. Nur auf diese Weise gelingt es, eine stabile Formulierung mit den oben genannten Vorteilen zu erhalten.

Das erfindungsgemäße Kosmetikum kann für Haare eingesetzt werden, insbesondere für Wimpernhaare und Augenbrauenfarben. Insbesondere kann es auch als temporäres Haarfärbemittel eingesetzt werden, das graue Strähnen abdeckt und einen verlängerten Nutzungskomfort bietet. Zu den typischen Nachteilen sogenannter wasserfester Mascara-Produkte des Marktes gehört die unbefriedigende Haltbarkeit nach dem Auftragen auf die Wimpern in Form von Wasserbeständigkeit und Hautölbeständigkeit. Das erfindungsgemäße Kosmetikum zeigt eine deutliche Verbesserung bei der Wasserfestigkeit im täglichen Gebrauch über einen längeren Zeitraum und eine zufriedenstellende Haut-ölbeständigkeit.

Bei der Anwendungsrichtung für die dekorative Kosmetik, also bei Lippenfarbe, Rougecreme, Grundierungen, Make-up, Lidschatten, Abdeckmischungen usw., wird ebenfalls eine deutliche Verbesserung gegenüber bekannten Produkten des Marktes erreicht, sowohl hinsichtlich der Abriebfestigkeit (bis zu 24 h) als auch der Wasserfestigkeit für einen längeren Zeitraum und unter unterschiedlichen Umgebungsbedingungen. Das heißt, auch ein Bad oder ein Duschvorgang oder ein Regen wird von dem erfindungsgemäßen Kosmetikum problemlos überstanden, wodurch es bekannten produkten deutlich überlegen ist.

Auch ein Klebrigkeitsgefühl, wie bei einer Reihe bekannter Produkte, wird vermieden. Das erfindungsgemäße Kosmetikum kann leicht aufgetragen und auch wieder gut entfernt werden und haftet ausgezeichnet auf der Haut und den Haaren.

Die Erfindung soll nachstehend durch Beispiele näher erläutert werden, die jedoch keine Einschränkung der Erfindung darstellen. Alle Angaben sind in Gewichtsprozent.

### Beispiel 1 Basisgel

| | |
|---|---|
| Ethyl Acrylate/Methyl Methacrylate | 24,5 |
| Wasser | 25,0 |
| Laureth-20 | 0,3 |
| Isododecane | 50,0 |

Das Copolymer wird Wasser und dem Emulgator Laureth-20 vermischt und das Gemisch auf 45°C erwärmt. Isododecan wird unter Rühren hinzugegeben und die Temperatur auf 50 °C erhöht. Danach erfolgt Homogenisieren mit 2200 U/min für eine Zeit von 18 Minuten in einem 1000 ml Laborbehälter. Dann wird das Gemisch auf 27 °C abgekühlt und dabei mit 400 U/min etwa 5 Min gerührt.

### Beispiel 2 Augenbrauenfarbe

| | |
|---|---|
| Carnauba Wax | 4,9 |
| Bees Wax | 3,3 |
| Castor Wax | 1,4 |
| Microcrystalline Wax | 5,0 |
| PVP/Eicosane Copolymer | 4,0 |
| Pigmente | 6,0 |
| Isododecane | 38,5 |
| Disteardimoninium Hectorite | 4,0 |
| Isododecane | 13,0 |
| Propylene Carbonate | 1,6 |
| Trimethylsiloxysilicate und Decamethylcyclopentasiloxane | 2,0 |
| Basisgel von Beispiel 1 | 16 |
| Konservierungsstoffe (Parabene) | 0,3 |

Die Wachsphase wurde bei 85°C geschmolzen. Dann wurden der Wachsphase Pigmente hinzugesetzt, und es wurde homogenisiert. Bei Aufrechterhaltung der Temperatur von etwa 60 °C wurde Isododecane hinzugegeben. Der mit Isododecane vorgemischte Ton wurde unter Rühren dem Gemisch zugesetzt. Die restlichen Bestandteile wurden bei etwa 40°C zugegeben, und es wurde homogenisiert.

### Beispiel 3 Mascara

| | |
|---|---|
| Carnauba Wax | 5,1 |
| Beeswax | 4,0 |
| Castor Wax | 1,9 |
| Paraffin | 6,0 |
| PVP/Eicosane Copolymer | 5,5 |
| Propylparaben | 0,2 |
| Pigmente | 8,0 |
| Petroleum Destillat | 45,0 |
| Disteardimoninium Hectorite | 3,0 |
| Petroleum Destillat | 12,0 |
| Propylene Carbonate | 1,2 |
| Basisgel von Beispiel 1 | 6,0 |
| Cylopentasiloxane (und) | |
| Trimethylsiloxysilicate | 2,1 |

Die Arbeitsweise erfolgte wie im Beispiel 2.

### Vergleichsbeispiel 4

Das Mascara von Beispiel 3 (Produkt A) wurde auf seine wasserfesten Eigenschaften und anderes nach Einschätzung durch Versuchspersonen getestet. Als Vergleich wurden handelsübliche Produkte bekannter Marken ausgewählt (Produkt B "... Volume Express WP", Produkt C "... Voluminous WP").

Die Bewertung erfolgte durch 23 Probanden auf einer Skala von 0 bis 5; 0 = schlechteste Bewertung, 5 = beste Bewertung.

| | Produkt A | Produkt B | Produkt C |
|---|---|---|---|
| Wasserbeständigkeit (4-18 h) | 4,1 | 3,7 | 3,4 |
| Vermeidung des Ausflockens | 3,7 | 3,2 | 3,4 |
| Vermeidung des Klumpens | 3,6 | 2,8 | 2,6 |
| Gesamtkomfort des Produktes während des Tragens | 4,4 | 3,6 | 3,1 |

Es zeigt sich eine deutliche Verbesserung für das erfindungsgemäße Produkt A, die im Mittel zwischen 15 und 30 % liegt.

### Beispiel 5 Halbmatte dauerhafte Lippenfarbe A

| | |
|---|---|
| Carnauba Wax | 4,9 |
| Bees Wax | 3,3 |
| Castor Wax | 1,4 |
| Microcrystalline Wax | 5,0 |
| PVP/Eicosane Copolymer | 4,0 |
| Pigmente | 5,0 |
| Isododecane | 40,5 |
| stearalkoninium Hectorite | 4,0 |
| Isododecane | 13,0 |
| Propylene Carbonate | 1,6 |
| PPG-51/SMDI Copolymer | 2,0 |
| Basisgel von Beispiel 1 | 16 |
| Konservierungsstoffe (Parabene) | 0,3 |

Die Arbeitsweise erfolgte wie im Beispiel 2.

### Beispiel 6 Rouge B

| | |
|---|---|
| Carnauba Wax | 4,9 |
| Bees Wax | 3,3 |
| Castor Wax | 1,4 |
| Microcrystalline Wax | 5,0 |
| PVP/Eicosane copolymer | 3,0 |
| Pigmente | 5,0 |
| Isododecane | 39,5 |
| Disteardimoninium Hectorite | 4,0 |
| Isododecane | 13,0 |
| Propylene Carbonate | 1,6 |
| Trimethylsiloxysilicate und Decamethylcyclopentasiloxane | 2,0 |
| Reisstärke | 1,0 |
| Basisgel von Beispiel 1 | 16 |
| Konservierungsstoffe (Parabene) | 0,3 |

Die Arbeitsweise erfolgte wie im Beispiel 2.

### Vergleichsbeispiel 7 Kuß-Test

Die Lippenfarbe von Beispiel 5 wurde von 12 Probanden auf ihre Lippen aufgetragen und 3 Minuten einwirken gelassen. Dann ließ man die Probanden ihren Handrücken küssen, und es wurde nach einer Bewertungsskala von 1 bis 5 (1 = nicht akzeptabel, 5 = ausgezeichnet) die Übertragung/der Abrieb eingeschätzt. Von den 12 Probanden schätzten 10 die Lippenfarbe A mit "5" ein, d.h. läßt nicht eine Spur von Farbe zurück". " Probanden schätzen mit "4" ein, d.h. "läßt eine Spur von Farbe zurück". Gleichwertige Produkte des Marktes zeigten um 10-15 % schlechtere Ergebnisse.

### Vergleichsbeispiel 8 Rouge-Test

Der Rouge-Test wurde nach Auftragen von Rouge gemäß Beispiel 7 auf die Wangen von 12 Probanden in ähnlicher Weise wie der Kuß-Test nach Vergleichsbeispiel 7 durchgeführt. Alle Probanden bewerteten mit "5". Gleichwertige Produkte des Marktes zeigten um 10-15 % schlechtere Ergebnisse.

## Patentansprüche

1. Wasser- und abriebfestes Kosmetikum für Haare und für dekorative Kosmetik, **dadurch gekennzeichnet, daß** es enthält
- 1-45 Gew-% eines stabilen Gemisches bestehend aus 0,01-30 Gew-% eines mit Wasser emulgierbaren, filmbildenden Mittels auf Basis eines Acrylatcopolymeren oder von Acryl- oder Methacrylsäure oder einem Ester davon; 0,01-35 Gew-% eines aliphatischen Kohlenwasserstoff-Lösungsmittels oder eines flüchtigen Siliconderivates, wobei beide mit Wasser nicht mischbar sind und mit auf Wasser basierenden Ingredienzen und/oder auf organischen Lösungsmitteln basierenden Ingredienzen in Gegenwart eines Emulgators emulgierbar sind;
0,01 - 2 Gew-% eines nicht-ionischen Emulgators; und 1 - 30 Gew-% Wasser,
und wobei diese Zusammensetzung die Struktur eines Gels hat und wobei alle Angaben auf das Gesamtgewicht des Kosmetikums bezogen sind;
- 1-45 Gew-% eines kosmetischen Wachses;
- 0,1-10 Gew-% eines in einem Lösungsmittel dispergierbaren rheologischen Modifiziermittels;
- 0,1-30 Gew-% einer Pigmentphase, umfassend mineralische Pigmente, organische Pigmente, Perlglanzpigmente, oberflächenbehandelte Pigmente und Gemische davon;
- 51-90 Gew-% eines flüchtigen organischen Lösungsmittels;
- 1-15 Gew-% eines öllöslichen polymeren Filmbildners;
- 0,01-30 Gew-% eines Siliconharzes;
- 0,01-20 Gew-% eines Füllstoffes;
wobei alle Angaben auf das Gesamtgewicht des Kosmetikums bezogen sind.

2. Kosmetikum nach Anspruch 1, **dadurch gekennzeichnet, daß** der Emulgator aus der Gruppe ausgewählt ist, bestehend aus alkoxylierten Alkoholen, ethoxylierten Alkoholen, Polyglyceryl Estern und Gemischen davon.

3. Kosmetikum nach Anspruch 1, **dadurch gekennzeichnet, daß** das filmbildende Mittel ein Acrylatcopolymeres ist.

4. Kosmetikum nach Anspruch 3, **dadurch gekennzeichnet, daß** das Acrylatcopolymere ein Ethylacrylat/Methylmethacrylat-Copolymers ist.

5. Kosmetikum nach Anspruch 3, **dadurch gekennzeichnet, daß** das filmbildende Mittel ein Ethylacrylat/Methylmethacrylat-Copolymeres mit einem Verhältnis vom Ethylacrylat-Einheiten zu Methylmethacrylat-Einheiten in dem Polymeren im Bereich von 7,5-8,5 : 1,8-2,3 ist.

6. Kosmetikum nach Anspruch 1, **dadurch gekennzeichnet, daß** es als eine Lippenfarbe, Rougecreme, Grundierung, Make-up, Lidschattencreme, Abdeckung, Augenbrauenfarbe, Mascara vorliegt.

7. Verwendung eines Kosmetikums gemäß Anspruch 1 als Lippenfarbe, Rougecreme, Grundierung, Make-up, Lidschattencreme, Abdeckung, Augenbrauenfarbe oder Mascara.

## Claims

1. A water- and abrasion- resistant cosmetic for hair and decorative cosmetics, **characterized in that** it contains:
- 1-45% by weight of a stable mixture consisting of 0.01-30% by weight of a film-forming agent which can be emulsified with water and is based on an acrylate copolymer or acrylic or methacrylic acid or an ester thereof; 0.01-35% by weight of an aliphatic hydrocarbon solvent or a volatile silicone derivative, wherein both are immiscible with water and can be emulsified with water-based ingredients and/or organic solvent-based ingredients in the presence of an emulsifying agent;
0.01-2% by weight of a non-ionic emulsifying agent; and 1-30% by weight of water;
and wherein this composition has a gel-like structure and wherein all of the figures are given with respect to the total weight of the cosmetic;
- 1-45% by weight of a cosmetic wax;
- 0.1-10% by weight of a rheology-modifying agent which can be dispersed in a solvent;
- 0.1-30% by weight of a pigment phase comprising mineral pigments, organic pigments, pearlescent pigments, surface-treated pigments and mixtures thereof;
- 51-90% by weight of a volatile organic solvent;
- 1-15% by weight of an oil-soluble polymeric film-forming agent;
- 0.01-30% by weight of a silicone resin;
- 0.01-20% by weight of a filler;
wherein all of the figures are given with respect to the total weight of the cosmetic.

2. The cosmetic according to claim 1, **characterized in that** the emulsifying agent is selected from the group consisting of alkoxylated alcohols, ethoxylated alcohols, polyglyceryl esters and mixtures thereof.

3. The cosmetic according to claim 1, **characterized in that** the film-forming agent is an acrylate copolymer.

4. The cosmetic according to claim 3, **characterized in that** the acrylate copolymer is an ethyl acrylate/methyl methacrylate copolymer.

5. The cosmetic according to claim 3, **characterized in that** the film-forming agent is an ethyl acrylate/methyl methacrylate copolymer with a ratio of ethyl acrylate units to methyl methacrylate units in the polymer in the range 7.5-8.5 : 1.8-2.3.

6. The cosmetic according to claim 1, **characterized in that** it is a lip colour, cream rouge, foundation, make-up, cream eye shadow, concealer, eyebrow colour or mascara.

7. Use of a cosmetic according to claim 1 as a lip colour, cream rouge, foundation, make-up, cream eye shadow, concealer, eyebrow colour or mascara.

## Revendications

1. Cosmétique résistant à l'eau et à l'abrasion destiné aux cheveux et au maquillage, **caractérisé en ce qu'**il contient
- 1 à 45 % en poids d'un mélange stable constitué de 0,01 à 30 % en poids d'un agent filmogène pouvant former une émulsion avec de l'eau, à base d'un copolymère d'acrylate ou à base d'acide acrylique ou d'acide méthacrylique ou de leurs esters ; 0,01 à 35 % en poids d'un solvant hydrocarboné aliphatique ou d'un dérivé de silicone volatil, les deux n'étant pas miscibles à l'eau et pouvant former, en présence d'un émulsifiant, une émulsion avec des ingrédients à base d'eau et/ou des ingrédients à base de solvants organiques ;
0,01 à 2 % en poids d'un émulsifiant non-ionique ; et 1 à 30 % en poids d'eau,
et cette composition ayant la structure d'un gel et l'ensemble des indications se rapportant au poids total dudit cosmétique ;
- 1 à 45 % en poids d'une cire à usage cosmétique ;
- 0,1 à 10 % en poids d'un agent de modification rhéologique pouvant être dispersé dans un solvant ;
- 0,1 à 30 % en poids d'une phase pigmentaire comprenant des pigments minéraux, des pigments organiques, des pigments nacrés, des pigments traités en surface et leurs mélanges ;
- 51 à 90 % en poids d'un solvant organique volatil ;
- 1 à 15 % en poids d'un agent filmogène polymère soluble dans l'huile ;
- 0,01 à 30 % en poids d'une résine de silicone ;
- 0,01 à 20 % en poids d'une charge ;
l'ensemble des indications se rapportant au poids total dudit cosmétique.

2. Cosmétique selon la revendication 1, **caractérisé en ce que** ledit émulsifiant est choisi dans le groupe constitué d'alcools alkoxylés, d'alcools éthoxylés, d'ester de polyglycéryle et de leurs mélanges.

3. Cosmétique selon la revendication 1, **caractérisé en ce que** l'agent filmogène est un copolymère d'acrylate.

4. Cosmétique selon la revendication 3, **caractérisé en ce que** ledit copolymère d'acrylate est un copolymère d'acrylate d'éthyle / méthacrylate de méthyle.

5. Cosmétique selon la revendication 3, **caractérisé en ce que** ledit agent filmogène est un copolymère d'acrylate d'éthyle / méthacrylate de méthyle, le rapport entre les unités d'acrylate d'éthyle et les unités de méthacrylate de méthyle au sein dudit polymère se situant dans la fourchette de 7,5 à 8,5 : 1,8 à 2,3.

6. Cosmétique selon la revendication 1, **caractérisé en ce qu'**il se présente sous forme d'un rouge à lèvres, d'un fard à joues, d'un fond de teint, d'un produit de maquillage, d'un fard à paupières, d'un correcteur, d'un crayon à sourcils, d'un mascara.

7. Utilisation d'un cosmétique selon la revendication 1 en tant que rouge à lèvres, fard à joues, fond de teint, produit de maquillage, fard à paupières, correcteur, crayon à sourcils ou mascara.
